# EUROPÄISCHE PATENTSCHRIFT

(11) **EP 3 819 444 B1**
(45) Veröffentlichungstag und Bekanntmachung des Hinweises auf die Patenterteilung: **22.12.2021**
(21) Anmeldenummer: 20202502.9
(22) Anmeldetag: 19.10.2020
(51) Int. Cl.: E05B 1/00, A61L 2/10

(54) **TÜRGRIFF**
DOOR HANDLE
POIGNÉE DE PORTE

(30) Priorität: 06.11.2019 DE 102019129863
(43) Veröffentlichungstag der Anmeldung: 12.05.2021
(73) Patentinhaber: Viebrans, Thomas, 78052 Villingen (DE)
(72) Erfinder: Viebrans, Thomas, 78052 Villingen (DE)
(74) Vertreter: Westphal, Mussgnug & Partner Patentanwälte mbB

(56) Entgegenhaltungen:
- DE-U1-202014 006 908
- JP-A- H07 327 763
- US-A1- 2007 145 292

## Beschreibung

Die Erfindung betrifft einen Türgriff.

Herkömmliche Türgriffe weisen ein Griffelement mit einer ersten Längsachse und ein Befestigungselement mit einer zweiten Längsachse auf, wobei die erste Längsachse und die zweite Längsachse in einem Winkel ungleich 0° zueinander verlaufen. Mittels des Befestigungselements kann der Türgriff an der Mechanik einer Tür befestigt werden, um die Tür zu öffnen und zu schließen. Ein Benutzer fasst üblicherweise an das Griffelement, um den Türgriff zu schwenken und dadurch ein Öffnen oder Schließen einer Tür zu bewirken.

Es ist bekannt, dass über Türgriffe Keime von einer Person auf eine andere Person übertragen werden. Weiterhin ist bekannt, dass UV-Licht, insbesondere UV-C-Licht, eine keimabtötende Wirkung aufweist. Die DE 20 2014 006 908 U1 offenbart bereits, eine Türklinke zur Desinfektion mit UV-C-Licht zu beaufschlagen. Die UV-C-Lichtquelle ist dabei in einem Tür-oder Fensterflügel angeordnet und bestrahlt den Türgriff. Nachteilig dabei ist, dass nicht auszuschließen ist, dass eine Person, beispielsweise ein kleines Kind, dessen Kopf auf Augenhöhe mit dem Türgriff ist, versehentlich in die UV-C-Lichtquelle blickt. Auch die DE 20 2015 001 059 U1 offenbart die Verwendung einer UV-Lichtquelle zur Entkeimung eines Türgriffs, wobei die UV-Lichtquelle im Inneren des Griffes angeordnet ist. Dabei können mehrere UV-LEDs in verschiedene Richtungen Licht abstrahlen. Um Personen nicht zu gefährden, sollen die Lichtpulse erst nach einigen Sekunden oder Minuten nach der Betätigung des Türgriffs erfolgen und ggfs. Anwesenheitsdetektoren vorgesehen werden. Nachteilig dabei ist, dass auch bei dieser Anordnung nicht vollständig auszuschließen ist, dass versehentlich eine Person in die UV-Lichtquelle blickt.

Weiterhin ist aus dem Dokument US 2007/145292 A1 ein Türgriff mit einem Griffelement bekannt, das einen für UV-Licht undurchlässigen Grundkörper aufweist. Der Grundkörper weist eine Wandung mit wenigstens einer Öffnung auf.

Die Aufgabe der Erfindung besteht daher darin, einen Türgriff bereitzustellen, welcher eine selbstdesinfizierende Funktion auf verbesserte Weise, insbesondere mit einer erhöhten Sicherheit, ermöglicht.

Die Aufgabe wird erfindungsgemäß gelöst durch einen Türgriff mit den Merkmalen des Patentanspruchs 1.

Vorteilhafte Ausgestaltungen und Weiterbildungen der Erfindung sind in den abhängigen Ansprüchen angegeben.

Der erfindungsgemäße Türgriff mit einem Griffelement mit einer ersten Längsachse und einem Befestigungselement mit einer zweiten Längsachse, wobei die erste Längsachse und die zweite Längsachse in einem Winkel ungleich 0° zueinander verlaufen, wobei das Griffelement einen für UV-Licht undurchlässigen Grundkörper aufweist, welcher eine Wandung mit wenigstens eine Öffnung aufweist, welche in einem an einer Tür montierten Zustand des Türgriffs in Richtung der Tür weist, wobei in dem Grundkörper eine UV-Lichtquelle angeordnet ist, und wobei auf der Außenseite des Grundkörpers eine Hülle aus für UV-Licht durchlässigem Material angeordnet ist, welche mittels einer Antriebseinheit um die erste Längsachse gegen den Grundkörper verdrehbar angeordnet ist.

Durch die Anordnung der UV-Lichtquelle in dem Türgriff in Verbindung mit der Tatsache, dass der Grundkörper die UV-Lichtquelle umschließt und die Öffnung, durch welche das UV-Licht aus dem Grundkörper austritt, in Richtung der Tür weisen, sodass die Öffnung vom Benutzer aus gesehen auf der rückwärtigen Seite des Griffelements liegt, kann verhindert werden, dass eine Person direkt in die Lichtquelle blicken kann. Vorzugsweise weist die Öffnung einen kleinen Öffnungswinkel auf, so dass Licht im Wesentlichen horizontal Richtung Tür fällt. Die Verdrehung der Hülle, welche außen angeordnet ist und somit das Element ist, welches in Kontakt mit dem Benutzer kommt, gegen den Grundkörper ermöglicht dabei jedoch eine Beaufschlagung der Hülle über den gesamten Umfang mit UV-Licht zur zuverlässigen Desinfektion der Hülle. Durch die Antriebseinheit kann eine automatisierte Desinfektion ermöglicht werden.

Es ist möglich, lediglich eine schlitzförmige Öffnung über den Großteil der Länge des Grundkörpers verlaufend vorzusehen. Ein derartiger Grundkörper kann jedoch an Stabilität einbüßen. Vorteilhafterweise weist daher der Grundkörper mehrere Öffnungen auf.

Besonders bevorzugt sind die Öffnungen derart angeordnet, dass bei einer vollständigen Umdrehung der Hülle gegen den Grundkörper jeder zwischen zwei gedachten Außenlinien angeordnete Punkt der Hülle mit durch die wenigstens eine Öffnung fallendem UV-Licht beaufschlagbar ist. Dadurch kann eine besonders zuverlässige Desinfektion des Türgriffs erreicht werden.

Eine vorteilhafte Weiterbildung der Erfindung sieht vor, dass die Öffnungen entlang wenigstens zwei parallel versetzt zueinander angeordneten Geraden in Längsrichtung zueinander beabstandet angeordnet sind. Dadurch können die Öffnungen platzsparend angeordnet werden.

Besonders bevorzugt ist eine Öffnung auf einer der Geraden mit zumindest einer der Öffnungen auf der anderen Geraden in Umfangsrichtung überlappend angeordnet, wodurch eine möglichst lückenlose Desinfektion des Türgriffs erreicht werden kann.

Als besonders geeignet zur Keimabtötung hat sich UV-L-Licht herausgestellt, so dass vorzugsweise die UV-Lichtquelle als UV-C-Lichtquelle ausgebildet ist.

Vorteilhafterweise ist die UV-Lichtquelle als UV-LED ausgebildet, welche insbesondere ohne eine Hochspannung auskommt.

Besonders bevorzugt wird als UV-Lichtquelle eine UV-C-LED verwendet.

Eine vorteilhafte Ausführungsform der Erfindung sieht vor, dass das für UV-Licht durchlässige Material Quarzglas ist.

Das von der UV-Lichtquelle ausgestrahlte Licht ist insbesondere dann zuverlässig Richtung Tür gerichtet, wenn die erste Längsachse und die zweite Längsachse eine Ebene aufspannen, und wenigstens eine Öffnung die Ebene schneidet.

Vorzugsweise weist die Antriebseinheit einen Motor und ein Zahnrad auf, wobei vorzugsweise der Motor in dem Grundkörper angeordnet ist und mit einer Gewindestange verbunden ist und das in die Gewindestange eingreifende Zahnrad mit der Hülle verbunden ist. Eine derartige Antriebseinheit kann kompakt ausgebildet sein und auf einfache Art und Weise in den Innenraum des Türgriffs integriert werden.

Der Türgriff weist vorzugsweise eine Stromversorgung auf, um die Antriebseinheit oder eventuell weitere vorhandene elektrische Komponenten mit Strom zu versorgen. Dazu ist besonders bevorzugt in dem Türgriff eine Batterie oder ein Akkumulator angeordnet, um einen Austausch gegen einen herkömmlichen Türgriff zu ermöglichen, bei welchem möglichst keine Änderungen an der Tür selbst vorgenommen werden müssen.

Eine besonders bevorzugte Ausführungsform der Erfindung sieht vor, dass in dem Türgriff ein Sensor, beispielsweise ein Näherungssensor, ein Beschleunigungssensor oder ein Bewegungssensor, angeordnet ist. Dieser kann ermitteln, ob eine Person sich dem Türgriff nähert oder den Türgriff berührt hat.

Vorzugsweise weist der Türgriff eine Steuerung auf, welche in Abhängigkeit von einem durch den Sensor detektierten Signal die UV-Lichtquelle und/oder die Antriebseinheit aktiviert. Dadurch wird ermöglicht, lediglich bedarfsweise den Desinfektionsprozess zu starten.

Ein Ausführungsbeispiel der Erfindung wird anhand der nachfolgenden Figuren detailliert erläutert. Es zeigen
- Fig. 1: eine perspektivische Ansicht eines Ausführungsbeispiels eines erfindungsgemäßen Türgriffs mit einem Griffelement und einem Befestigungselement,
- Fig. 2: eine weitere perspektivische Ansicht des Türgriffs gemäß Fig. 1,
- Fig. 3: eine perspektivische Ansicht des Grundkörpers des Türgriffs gemäß Fig. 1 mit darin angeordneter UV-Lichtquelle und Antriebseinheit,
- Fig. 4: eine Ansicht von vorne auf den Grundkörper gemäß Fig. 4,
- Fig. 5: eine perspektivische Ansicht der Antriebseinheit und eines Abschlussdeckels für den Grundkörper des Türgriffs gemäß Fig. 1,
- Fig. 6: den Abschlussdeckel mit Antriebseinheit gemäß Fig. 5 mit daran angeordneter Hülle und
- Fig. 7: eine perspektivische Ansicht des Befestigungselements des Türgriffs gemäß Fig. 1.

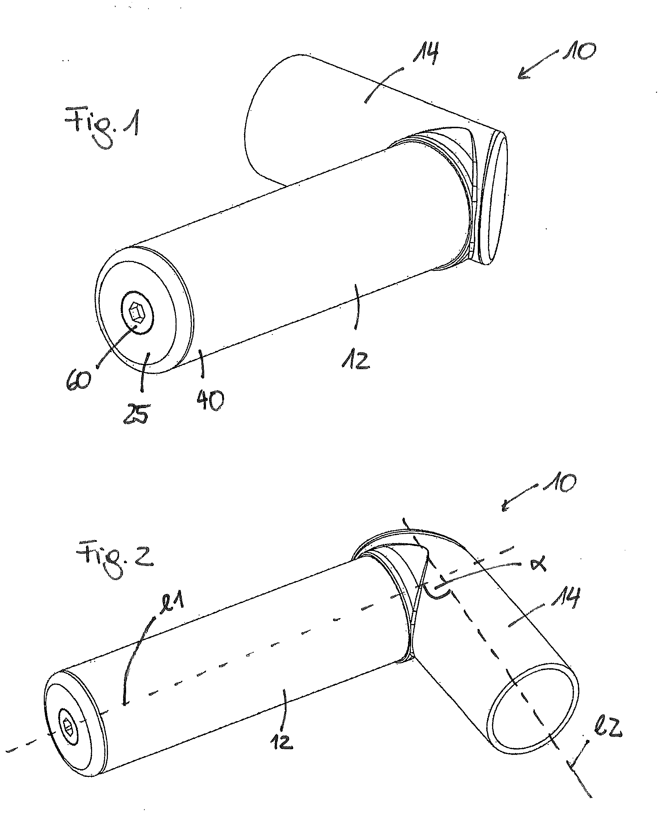

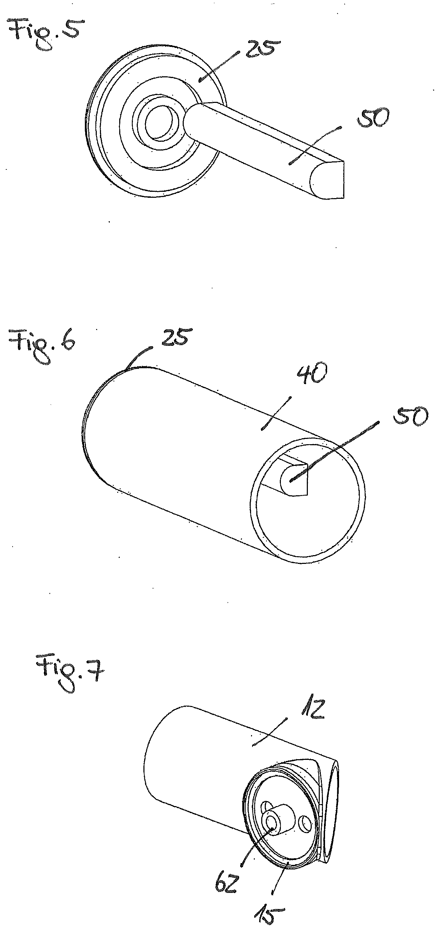

Die Figuren 1 bis 7 zeigen verschiedene Ansichten, teils von unterschiedlichen Komponenten eines Ausführungsbeispiels eines erfindungsgemäßen Türgriffs 10 mit einem Griffelement 12 und einem Befestigungselement 14. Gleiche Bezugsziffern bezeichnen gleiche Teile, wobei zur besseren Übersicht nicht sämtliche Bezugsziffern in sämtlichen Figuren angegeben sind.

Das Griffelement 12 weist eine erste Längsachse 11 auf, das Befestigungselement weist eine zweite Längsachse 12 auf, wobei die erste Längsachse 11 und die zweite Längsachse 12 in einem Winkel α, welcher ungleich 0° ist, und insbesondere etwa 90° beträgt, zueinander angeordnet sind. Die erste Längsachse 11 und die zweite Längsachse 12 spannen eine Ebene auf, die in einem an einer Tür montierten Zustand des Türgriffs und nicht betätigtem Türgriff in etwa horizontal verlaufen kann.

Mittels des Befestigungselements 14 kann der Türgriff an einer Tür, insbesondere der Mechanik der Tür, befestigt werden, um durch Betätigen des Türgriffs, insbesondere Verschwenken des Türgriffs, ein Öffnen oder Schließen der Tür bewirken zu können. Das Befestigungselement 14 ist insbesondere derart ausgebildet, dass es an standardisierten Mechaniken angeordnet werden kann, wozu es beispielsweise eine Ausnehmung zur Aufnahme eines Vierkants und eine Madenschraube zur Fixierung aufweisen kann.

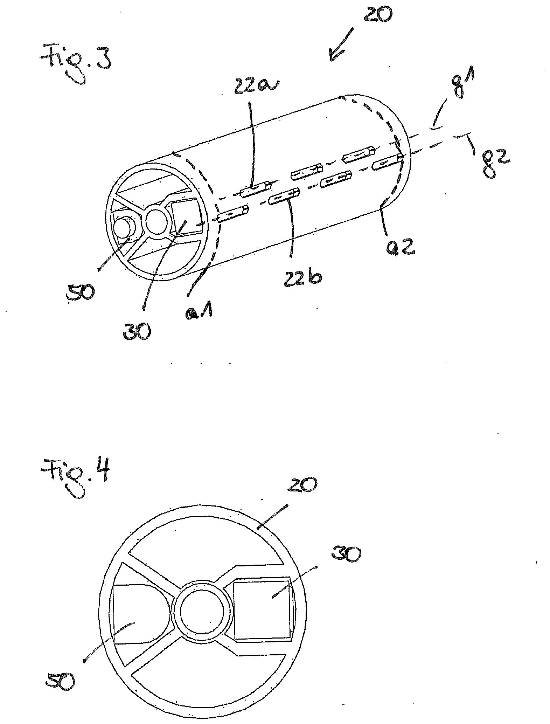

Das Griffelement 12 weist einen Grundkörper 20 auf, welcher für UV-Licht undurchlässig ist und dazu entweder aus einem für UV-Licht undurchlässigen Material gefertigt ist oder eine für UV-Licht undurchlässige Beschichtung aufweist. Der Grundkörper 20 ist vorzugsweise zylindrisch, insbesondere kreiszylindrisch, ausgebildet. Der Grundkörper 20 ist als Hohlkörper mit einer Wandung ausgebildet, in welcher wenigstens eine Öffnung 22, vorzugsweise mehrere Öffnungen 22 angeordnet sind (vgl. Fig. 3). Die Öffnungen 22 weisen in einem an einer Tür montierten Zustand des Türgriffs 10 in Richtung Tür. Dadurch liegen die Öffnungen 22 vom Benutzer aus gesehen auf der rückwärtigen Seite des Griffelements. Insbesondere schneidet wenigstens eine Öffnung die durch die erste Längsachse 11 und die zweite Längsachse 12 aufgespannte Ebene. Vorzugsweise weisen die Öffnungen 22 einen kleinen Öffnungswinkel auf, so dass das UV-Licht in einem schmalen Lichtspalt im Wesentlichen senkrecht auf die Tür trifft.

Im Innern des Grundkörpers 20 ist eine UV-Lichtquelle 30 angeordnet, welche insbesondere als UV-C-Lichtquelle ausgebildet ist. Die UV-Lichtquelle ist dabei insbesondere als UV-LED, besonders bevorzugt als UV-C-LED, ausgebildet. Die UV-Lichtquelle ist derart angeordnet, dass von ihr ausgesandtes Licht durch die Öffnungen 22 nach außen dringen kann. Vorzugsweise erstreckt sich die UV-Lichtquelle im Wesentlichen über nahezu die ganze Länge des Grundkörpers.

Auf der Außenseite des Grundkörpers 20 ist eine Hülle 40 aus für UV-Licht durchlässigem Material, beispielsweise aus Quarzglas, angeordnet ist, welche um die erste Längsachse 11 gegen den Grundkörper 20 verdrehbar angeordnet ist. Die Hülle 40 ist insbesondere als Hohlrohr ausgebildet.

Die Öffnungen 22 des Grundkörpers 20 sind insbesondere derart angeordnet, dass bei einer vollständigen Umdrehung der Hülle 40 gegen den Grundkörper 20 jeder zwischen zwei gedachten Außenlinien a1, a2 angeordnete Punkt der Hülle 40 mit durch die Öffnungen 22 fallendem UV-Licht beaufschlagbar ist. Die Außenlinien a1, a2 können beispielsweise mit den Stirnkanten des Hohlrohres zusammenfallen oder verlaufen in möglichst kleinem Abstand zu diesen.

Die Öffnungen 22 können entlang wenigstens zweier, im dargestellten Ausführungsbeispiel entlang genau zweier, parallel versetzt zueinander angeordneten Geraden g1, g2 zueinander beabstandet angeordnet sein, wie insbesondere in Figur 3 erkennbar. Dabei kann eine Öffnung 22 auf einer der Geraden g1, g2 - beispielsweise die Öffnung 22a auf der Geraden g1 - mit zumindest einer der Öffnungen 22 auf der anderen Geraden g2, g1 - beispielsweise die Öffnung 22b auf der Geraden g2 - in Umfangsrichtung überlappend angeordnet sein. Die Außenlinien a1 und a2 verlaufen bei diesem Ausführungsbeispiel umlaufend durch jeweils den zu den Stirnseiten des Grundkörpers 20 am nächsten liegenden Rand der entsprechenden Öffnung 22 projiziert nach außen auf die Hülle 40.

Der Grundkörper 20 kann mittels einer Schraube 60 , die stirnseitig in den Grundkörper 20 eingeführt ist und den Grundkörper über die gesamte Länge durchsetzt, an dem Befestigungselement 12 befestigt werden, indem die Schraube 60 in eine an dem Befestigungselement 12 angeordnete Gewindeausnehmung 62 eingedreht wird. Die dem Befestigungselement 12 abgewandte Stirnseite des Grundkörpers 20 kann durch einen Abschlussdeckel 25 verschlossen sein, welcher insbesondere durch die Schraube 60 an dem Grundkörper 20 fixiert ist.

Die Hülle 40 umschließt die Außenwandung des Grundkörpers 20 und kann seitlich beispielsweise durch den Abschlussdeckel 25 und eine entsprechende Führungsfläche 15 des Befestigungselements geführt gehalten sein. Die Hülle 40 ist dabei um den Grundkörper 20, insbesondere dessen erste Längsachse 11, verdrehbar gelagert.

Der Türgriff 10 weist eine Antriebseinheit 50 zum Verdrehen der Hülle 40 gegen den Grundkörper 20 auf. Die Antriebseinheit ist beispielsweise in dem Grundkörper 50 angeordnet. Die Antriebseinheit 50 kann beispielsweise einen Motor, eine Gewindestange und ein Zahnrad aufweisen, wobei der Motor die Gewindestange dreht. Der Motor und die Gewindestange sind vorzugsweise mit dem Grundkörper 20 drehfest verbunden, während das Zahnrad mit der Hülle 40 drehfest verbunden ist. Dazu kann beispielsweise das Zahnrad umlaufend auf der Innenfläche der Hülle 40 angeordnet sein. Alternativ kann die Hülle 40 drehfest mit dem Abschlussdeckel 25 verbunden und das Zahnrad auf der Innenfläche der Abschlussdeckels angeordnet sein. Das Zahnrad greift in die Gewindestange ein, wodurch eine Drehung der Hülle 40 um die erste Längsachse bewirkt wird, wenn der Motor die Gewindestange dreht.

Dreht sich die Hülle 40 relativ zu dem Abschlussdeckel 25, kann die Länge der Hülle 40 geringfügig kleiner ausgebildet sein als die Länge des Grundkörpers 20, um Reibungen bei der Drehbewegung zwischen der Hülle 40 und dem Abschlussdeckel 25 zu verringern oder zu vermeiden.

Der Türgriff 10 weist eine Stromversorgung auf. Dazu kann beispielsweise in dem Türgriff 10, vorzugsweise in dem Befestigungselement 12, eine Batterie oder ein Akkumulator angeordnet sein. Diese dient zur Stromversorgung der Antriebseinheit, der UV-Lichtquelle und gegebenenfalls weiterer elektrischer Komponenten.

Beispielsweise kann der Türgriff einen Sensor wie beispielsweise einen Näherungssensor, einen Beschleunigungssensor oder einen Bewegungssensor aufweisen, um den Kontakt einer Person mit dem Türgriff oder auch bereits ein Annähern einer Person an den Türgriff detektieren zu können. Zusätzlich kann der Türgriff eine Steuerung aufweisen, welche in Abhängigkeit von einem durch den Sensor detektierten Signal die UV-Lichtquelle und/oder die Antriebseinheit aktiviert. Damit wird es möglich, einen Desinfektionsvorgang zu starten, wenn ein Kontakt des Türgriffs durch eine Person mittels des Sensors detektiert wurde. Zum Desinfektionsvorgang wird die UV-Lichtquelle eingeschaltet und die Antriebseinheit derart aktiviert, dass eine vollständige Umdrehung der Hülle 40 um die erste Längsachse 11 durchgeführt wird. Dadurch wird bei Anordnung der Öffnungen 22 wie in dem Ausführungsbeispiel dargestellt jeder Punkt der Hülle 40 zwischen den beiden Außenlinien a1, a2 mit UV-Licht, welches durch die Öffnungen 22 fällt, beaufschlagt und die Hülle 40 zuverlässig entkeimt.

### Bezugszeichenliste

- 10: Türgriff
- 12: Griffelement
- 14: Befestigungselement
- 15: Führungsfläche
- 20: Grundkörper
- 22: Öffnung
- 22a: Öffnung
- 22b: Öffnung
- 25: Abschlussdeckel
- 30: UV-Lichtquelle
- 40: Hülle
- 50: Antriebseinheit
- 60: Schraube
- 62: Gewindeausnehmung
- 11: erste Längsachse
- 12: zweite Längsachse
- g1: erste Gerade
- g2: zweite Gerade
- a1: erste Außenlinie
- a2: zweite Außenlinie
- α: Winkel

## Patentansprüche

1. Türgriff (10) mit einem Griffelement (12) mit einer ersten Längsachse (11) und einem Befestigungselement (14) mit einer zweiten Längsachse (12), wobei die erste Längsachse (11) und die zweite Längsachse (12) in einem Winkel (α) ungleich 0° zueinander verlaufen, wobei das Griffelement (12) einen für UV-Licht undurchlässigen Grundkörper (20) aufweist, welcher eine Wandung mit wenigstens einer Öffnung (22) aufweist, welche in einem an einer Tür montierten Zustand des Türgriffs (10) in Richtung der Tür weist, wobei in dem Grundkörper (20) eine UV-Lichtquelle (30) angeordnet ist, und wobei auf der Außenseite des Grundkörpers (20) eine Hülle (40) aus für UV-Licht durchlässigem Material angeordnet ist, welche mittels einer Antriebseinheit (50) um die erste Längsachse (11) gegen den Grundkörper (20) verdrehbar angeordnet ist.

2. Türgriff nach Anspruch 1,
**dadurch gekennzeichnet, dass** der Grundkörper (20) mehrere Öffnungen (22) aufweist.

3. Türgriff nach Anspruch 1 oder 2,
**dadurch gekennzeichnet, dass** die Öffnungen (22) derart angeordnet sind, dass bei einer vollständigen Umdrehung der Hülle (40) gegen den Grundkörper (20) jeder zwischen zwei gedachten Außenlinien (a1, a2) angeordnete Punkt der Hülle (40) mit durch die wenigstens eine Öffnung (22) fallendem UV-Licht beaufschlagbar ist.

4. Türgriff nach einem der vorhergehenden Ansprüche,
**dadurch gekennzeichnet, dass** die Öffnungen (22) entlang wenigstens zwei parallel versetzt zueinander angeordneten Geraden (g1, g2) in Längsrichtung zueinander beabstandet angeordnet sind.

5. Türgriff nach einem der vorhergehenden Ansprüche,
**dadurch gekennzeichnet, dass** eine Öffnung (22, 22a) auf einer der Geraden (g1, g2) mit zumindest einer der Öffnungen (22, 22b) auf der anderen Geraden (g1, g2) in Umfangsrichtung überlappend angeordnet ist.

6. Türgriff nach einem der vorhergehenden Ansprüche,
**dadurch gekennzeichnet, dass** die UV-Lichtquelle (30) als UV-C-Lichtquelle ausgebildet ist.

7. Türgriff nach einem der vorhergehenden Ansprüche,
**dadurch gekennzeichnet, dass** die UV-Lichtquelle (30) als UV-LED ausgebildet ist.

8. Türgriff nach einem der vorhergehenden Ansprüche,
**dadurch gekennzeichnet, dass** das für UV-Licht durchlässige Material Quarzglas ist.

9. Türgriff nach einem der vorhergehenden Ansprüche,
**dadurch gekennzeichnet, dass** die erste Längsachse (11) und die zweite Längsachse (12) eine Ebene aufspannen, und wenigstens eine Öffnung (22) die Ebene schneidet.

10. Türgriff nach einem der vorhergehenden Ansprüche,
**dadurch gekennzeichnet, dass** die Antriebseinheit (50) einen Motor und ein Zahnrad aufweist, wobei vorzugsweise der Motor in dem Grundkörper (20) angeordnet ist und mit einer Gewindestange verbunden ist und das in die Gewindestange eingreifende Zahnrad mit der Hülle (40) verbunden ist.

11. Türgriff nach einem der vorhergehenden Ansprüche,
**dadurch gekennzeichnet, dass** in dem Türgriff (10) eine Batterie oder ein Akkumulator angeordnet ist.

12. Türgriff nach einem der vorhergehenden Ansprüche,
**dadurch gekennzeichnet, dass** in dem Türgriff (10) ein Sensor, beispielsweise ein Näherungssensor, ein Beschleunigungssensor oder ein Bewegungssensor, angeordnet ist.

13. Türgriff nach einem der vorhergehenden Ansprüche,
**dadurch gekennzeichnet, dass** der Türgriff (10) eine Steuerung aufweist, welche in Abhängigkeit von einem durch den Sensor detektierten Signal die UV-Lichtquelle (30) und/oder die Antriebseinheit (50) aktiviert.

## Claims

1. Door handle (10) comprising a handle element (12) with a first longitudinal axis (11) and a fastening element (14) with a second longitudinal axis (12), the first longitudinal axis (11) and the second longitudinal axis (12) running at an angle (α) unequal to 0° to one another, the handle element (12) comprising a base body (20) which is impermeable to UV light and which comprises a wall with at least one opening (22) which, in a state of the door handle (10) mounted on a door, points in the direction of the door, a UV light source (30) being arranged in the base body (20), and an envelope (40) made of material which is permeable to UV light being arranged on the outside of the base body (20), the envelope (40) being arranged such that it can be rotated about the first longitudinal axis (11) with respect to the base body (20) by means of a drive unit (50).

2. Door handle according to claim 1,
**characterised in that** the base body (20) comprises several openings (22).

3. Door handle according to claim 1 or 2,
**characterised in that** the openings (22) are arranged in such a way that, during a complete rotation of the envelope (40) against the base body (20), each point of the envelope (40) arranged between two imaginary outer lines (al, a2) can be exposed to UV light falling through the at least one opening (22).

4. Door handle according to any one of the preceding claims, **characterised in that** the openings (22) are arranged at a distance from one another in the longitudinal direction along at least two straight lines (gl, g2) arranged parallel to and offset from one another.

5. Door handle according to any one of the preceding claims, **characterised in that** an opening (22, 22a) on one of the straight lines (gl, g2) is arranged overlapping with at least one of the openings (22, 22b) on the other straight line (gl, g2) in circumferential direction.

6. Door handle according to any one of the preceding claims, **characterised in that** the UV light source (30) is designed as a UV-C light source.

7. Door handle according to any one of the preceding claims, **characterised in that** the UV light source (30) is designed as a UV LED.

8. Door handle according to any one of the preceding claims, **characterised in that** the material permeable to UV light is quartz glass.

9. Door handle according to any one of the preceding claims, **characterised in that** the first longitudinal axis (11) and the second longitudinal axis (12) span a plane, and at least one opening (22) intersects the plane.

10. Door handle according to any one of the preceding claims, **characterised in that** the drive unit (50) comprises a motor and a cogwheel, preferably the motor being arranged in the base body (20) and being connected to a threaded rod and the cogwheel engaging the threaded rod being connected to the envelope (40).

11. Door handle according to any one of the preceding claims, **characterised in that** a battery or an accumulator is arranged in the door handle (10).

12. Door handle according to any one of the preceding claims, **characterised in that** a sensor, for example a proximity sensor, an acceleration sensor or a movement sensor, is arranged in the door handle (10).

13. Door handle according to any one of the preceding claims, **characterised in that** the door handle (10) comprises a control which activates the UV light source (30) and/or the drive unit (50) depending on a signal detected by the sensor.

## Revendications

1. Poignée de porte (10) comportant un élément de préhension (12) ayant un premier axe longitudinal (11) et un élément de fixation (14) ayant un second axe longitudinal (12),
le premier axe longitudinal (11) et le second axe longitudinal (12) se coupant suivant un angle (α) différent de 0°,
- l'élément de préhension (12) ayant un corps de base (20) opaque à la lumière UV, qui comporte une paroi avec au moins une ouverture (22) qui, à l'état installé de la poignée (10) sur une porte, est orienté vers la porte,
* le corps de base (20) ayant une source de lumière UV (30), et
* le côté extérieur du corps de base (20) étant muni d'une enveloppe (40) en une matière transparente à la lumière UV, et qui est montée à rotation par une unité d'entrainement (50) autour de l'axe longitudinal (11) par rapport au corps de base (20).

2. Poignée de porte (10) selon la revendication 1,
**caractérisée en ce que**
le corps de base (20) comporte plusieurs ouvertures (22).

3. Poignée de porte (10) selon la revendication 1 ou 2,
**caractérisée en ce que**
les ouvertures (22) sont disposées de façon que pour une rotation complète de l'enveloppe (40) par rapport au corps de base (20), chaque point de l'enveloppe (40) compris entre deux lignes extérieures géométriques (a1, a2) reçoit de la lumière UV traversant au moins une ouverture (22).

4. Poignée de porte (10) selon l'une des revendications précédentes,
**caractérisée en ce que**
les ouvertures (22) sont décalées l'une par rapport à l'autre sur au moins deux droites parallèles (g1, g2), décalées, orientées selon des droites (g1, g2) parallèles dans la direction longitudinale.

5. Poignée de porte (10) selon l'une des revendications précédentes,
**caractérisée en ce que**
une ouverture (22, 22a) est prévue sur l'une des droites (g1, g2) avec au moins l'une des ouvertures (22, 22b) situées sur l'autre droite (g1, g2) et se chevauchant dans la direction périphérique.

6. Poignée de porte (10) selon l'une des revendications précédentes,
**caractérisée en ce que**
la source de lumière UV (30) est une source de lumière UV-C.

7. Poignée de porte (10) selon l'une des revendications précédentes,
**caractérisée en ce que**
la source de lumière UV (30) est une source de lumière UV-LED.

8. Poignée de porte (10) selon l'une des revendications précédentes,
**caractérisée en ce que**
la matière transparente à la lumière UV est du verre de quartz.

9. Poignée de porte (10) selon l'une des revendications précédentes,
**caractérisée en ce que**
le premier axe longitudinal (11) et le second axe longitudinal (12) soustendent un plan et au moins une ouverture (22) coupe le plan.

10. Poignée de porte (10) selon l'une des revendications précédentes,
**caractérisée en ce que**
l'unité d'entrainement (50) comprend un moteur et une roue dentée,
de préférence le moteur est dans le corps de base (20) et il est relié à une tige filetée et la roue dentée en prise dans la tige filetée est reliée au manchon (40).

11. Poignée de porte (10) selon l'une des revendications précédentes,
**caractérisée en ce que**
la poignée de porte (10) loge une batterie ou un accumulateur.

12. Poignée de porte (10) selon l'une des revendications précédentes, **caractérisée en ce que**
la poignée de porte (10) comporte un capteur par exemple un capteur d'approche, un capteur d'accélération ou un capteur de mouvement.

13. Poignée de porte (10) selon l'une des revendications précédentes, **caractérisée en ce que**
la poignée de porte (10) comporte une commande qui en fonction du signal détecté par un capteur, active la source de lumière UV (30) et/ou l'unité d'entrainement (50).
